(19) Europäisches Patentamt

European Patent Office

Office européen des brevets

(11) **EP 1 114 992 B1**

(12) **EUROPEAN PATENT SPECIFICATION**

(45) Date of publication and mention
of the grant of the patent:
**25.05.2005 Bulletin 2005/21**

(51) Int Cl.⁷: **G01N 1/24**, G01N 33/20,
G01N 33/00

(21) Application number: **00311501.1**

(22) Date of filing: **20.12.2000**

(54) **Device and method for hydrogen collection and detection**

Anordnung und Verfahren zum Sammeln und Feststellen von Wasserstoff

Dispositif et méthode pour recueillir et détecter de l'hydrogène

(84) Designated Contracting States:
**AT BE CH CY DE DK ES FI FR GB GR IE IT LI LU MC NL PT SE TR**

(30) Priority: **05.01.2000 GB 0000016**

(43) Date of publication of application:
**11.07.2001 Bulletin 2001/28**

(73) Proprietor: **Ion Science Limited**
**Fowlmere, Cambridge SG8 7UJ (GB)**

(72) Inventors:
  • **Dean, Frank William Houlton**
    **Cambridge CB1 2LG (GB)**
  • **Ling, Alan Edward**
    **Foxton, Cambridge CB2 6SP (GB)**

(74) Representative: **Messulam, Alec Moses et al**
**A. Messulam & Co. Ltd.,**
**43-45 High Road**
**Bushey Heath, Herts WD23 1EE (GB)**

(56) References cited:
**GB-A- 2 312 279**        **US-A- 2 660 053**
**US-A- 4 820 920**        **US-A- 5 279 169**
**US-A- 5 517 026**

**Description**

This invention is concerned with hydrogen collection and detection. More specifically, the invention relates to the examination of iron and steel (and perhaps other materials) to determine whether they are giving off hydrogen gas, and so may be at risk from such problems as hydrogen embrittlement, and to devices for use in such an examination.

Hydrogen is often inadvertently introduced to steel at high temperatures, such as in a melt, or during welding in a damp atmosphere. The solubility of hydrogen in steel increases with temperature, so these processes may result in supersaturation of hydrogen when the metal cools. Supersaturation of hydrogen in steel may also occur upon corrosion, electroplating, and other surface treatments, or upon exposure of the steel surface to hydrogen sulphide ($H_2S$; sour gas, as often found in oil deposits, and thus in the pipelines carrying the oil and its derivatives elsewhere). Moreover, chemical reactions effected on an industrial scale are often carried out in steel vessels and pipework, and those that involve hydrogen and high temperatures and pressures, such as the hydrogenation of alkenes, can result in deleterious hydrogen absorption by the steel.

In mild steels a large proportion of the hydrogen is free to diffuse through the steel, emanating at the steel surface. The measurement of this diffusible hydrogen in steel is very important, since the hydrogen embrittles the steel, lowering its tensile strength, and may ultimately cause the steel to blister or crack. These deleterious effects may only be apparent many days after the hydrogen was introduced. A knowledge of the hydrogen in such a material is therefore of considerable value, and the measurement of a hydrogen flux (flow per unit area) emanating from a steel surface provides an indication of this, allowing, first and foremost, a means of quality control at various stages of steel processing and deployment. If the hydrogen flux is high, appropriate measures can be taken before the hydrogen leads to some mischief, such as a cracked steel pipeline or an embrittled weld.

In the Specification of our British Patent No: 2,312,279 (P1448) there is described and claimed a hydrogen detection system. This system is defined as one for detecting the hydrogen emanating from the surface of a solid, the system comprising:

walls defining an open enclosure the opening to which is in operation to be closed by the surface to be sensed for emanating hydrogen, which enclosure is for gathering into a carrier gas passed therethrough hydrogen emanating from the sensed solid surface, the enclosure having an input means for receiving the carrier gas and output means for delivering the resultant mixture of carrier gas and emanated hydrogen; and
a hydrogen detector connected to the enclosure output means, to receive the mixture of carrier gas and emanated hydrogen therefrom.

In operation, the enclosure - hereinafter referred to as the "collector" - is pressed against the surface from which hydrogen is thought to be emanating, and carrier gas is then drawn into the formed closed volume, and, taking any hydrogen with it, is passed on to the actual hydrogen detector apparatus.

The present invention also concerns such a hydrogen collection and detection system, likewise employing a carrier gas, preferably ambient air, to collect within an enclosure hydrogen emanating from the test surface, and convey it to some remote hydrogen gas sensor. However, the present invention relates specifically to the physical nature of the collector, and to the manner in which it can conveniently be attached to the test surface even when that surface is of a non-planar form.

The system of the aforementioned Patent is satisfactory for use in connection with flat surfaces, to which attachment of a collector can be carefully assured. However, there is a need for the convenient attachment and detachment of a collector to surfaces that are not flat - curved surfaces such as those of a pipe - and it is obviously desirable that the collector be able to be attached properly to surfaces having a curvature falling within quite a wide range. This will be better understood from a consideration of the following.

Due to the manner in which it is introduced, hydrogen may emanate from a steel (say) surface only in localised 'hotspots', and it may emanate over infrequent and unpredictable periods of time. For example, in gas pipelines conveying oil volatiles, sour gas and water, hydrogen entry may be confined to 'puddles' of water at the base of the pipe's interior. Eventually this hydrogen will exit at the pipe's exterior face, but that may not be until after a delay of several days (indeed, the delay may vary - for example, in consequence of variable composition of the interior gas - over a timescale ranging from days to years). In another example, in either routine or multipass welding only an occasional weld may contain significant diffusible hydrogen due to a damp weld consumable. And in a further example, hydrogen may have been introduced into a cast steel only from an occasional batch of molten steel which contained dissolved hydrogen sourced from one particular feedstock ingredient. It is therefore frequently necessary to carry out multiple tests on steel surfaces in the search for a prospective hydrogen flux, over a period of time, or over a range of surfaces. These steel surfaces may be of variable surface geometry either on account of unpredictable factors, as is the case with steel plate that is milled to a flatness tolerance of no better than a few millimetres per metre length, or due to predictable factors, as is the case with steel pipes in a production plant whose diameters commonly vary by as much as from 10 to 100cm.

Of course, to be both meaningful and useful any hydrogen flux measurement must be reproducible. Thus, intermittent testing for flux from a pipe surface over several months may only provide an insight into progressive enhancement or attenuation of hydrogen entry at the pipe's interior if the measurements are comparable - in other words, if they provide effective monitoring of the hydrogen flux over significantly long time periods. Also, it is usually desirable and frequently possible to assign for a given steel and hydrogen entry regime a critical flux at which hydrogen embrittlement is a possibility and where some remedial action should be undertaken.

The present invention seeks first to deal with the conformity problem - having the collector flexible enough to "fit" the surface to which it is applied regardless, within reason, of how much or little it is curved. The problem is solved by a collector device and a method of utilizing it as defined in the appended claims. More particularly, to render the collector conformable to surfaces of moderate curvature the invention proposes making it as a flexible (and preferably an elastically-flexible) plate on the receptor face of which are constructed raised grooves or high spots, which may be presented approximately parallel to an approximately flat surface or approximately tangential to a curved surface, and which form guiding channels along which hydrogen emanating from the surface underlying the plate can be drawn and sent to the detector system.

In one aspect, therefore, this invention provides, for use as the collector of a hydrogen collection and detection system, a flexible plate on the receptor face of which are constructed guide channels along which in use hydrogen emanating from the surface underlying the plate can be drawn and sent to the associated detector system.

The invention also provides such a collector plate that can be used to form the enclosure the subject of the aforementioned Patent, and which therefore has input means for receiving a carrier gas and output means for delivering the resultant mixture of carrier gas and emanated hydrogen, and which is operatively connected to a hydrogen detector for reception of the mixture of carrier gas and emanated hydrogen collected thereby and for the detection and measurement thereof.

The collector of this invention is a flexible plate. The plate material can be any that is sufficiently flexible - and, in particular, elastically (rather than plasticly) flexible, so that it will recover its own, basic, shape without the need for any intervention - to allow the plate to bend to the shape of the surface against which it is to be used but which will not allow the collected hydrogen to diffuse therethrough (and so be lost before it can be detected and measured). The plate body (as distinct from the high spots defining the channel) is most preferably made of a metal, a metallised plastic, or a plastic/metal laminate. "Bare" elastomeric plastics, some of which are highly elastically deformable, have been tested, but are generally found to be too permeable to hydrogen, so reducing the effective hydrogen capture area significantly, and hence raising the minimum flux detection threshold. Even if a plastic were found for which the hydrogen permeability were adequately low in this regard, significant outgassing of hydrogen might occur in a second test subsequent to permeation of hydrogen into the plastic in a first test (for instance, this might happen immediately following a test on a surface zone of high emanating flux) such that during a test at another site of zero flux sufficient from the plastic could be entrained into the gas stream to give rise to a false indication of hydrogen flux emanating from the latter site. The preferred material is therefore metal, the dimensions - and specifically the thickness - of the plate being such as to afford it the desired flexibility. Stainless steel is a good metal for this purpose, being particularly tough and sufficiently flexible to be most suitable.

The gas is gathered into, and swept along, channels formed in the receptor surface of the plate, and that face carries high spots that are effectively walls that define the channels. Efficient sweeping of gas from the space confined between the test surface, the plate face and the high spots necessitates limited plate contact with the test surface, so that the grooves walls/high spots are inevitably under considerable compression when the plate is up against the test surface. Plastics and plastic laminates are more likely to be damaged by rough contact with the test surface than metal, typically when the collector is inadvertently dragged across the surface by the operator. However, in certain applications, a tough, flexible and elastically-deformable groove wall made of a deformable plastic is preferential in ensuring a good seal between the plate/steel contact surface, and therefore increasing the reproducibility of measurement.

Most preferably, though, the plate is comprised entirely of sheet stainless steel into which are formed the collecting grooves, and desirably the steel is not tempered, so as to retain its flexibility characteristics. One very convenient way to form the required grooves is by chemical etching; leaving the wall portions behind, this provides a practical and inexpensive plate with sufficient toughness to endure many tests on even rough surfaces (as commonly encountered when testing, for instance, a steel pipeline pipe for the conveyance of gas sourced from sour oil).

Clearly, the collector plate of the invention may be of any size appropriate to its intended purpose. For attachment to a surface of a curvature corresponding to that of a cylindrical surface - like a pipeline pipe - of from 30cm (about lft) diameter and above, a plate of 15cm (about 6in) diameter is generally satisfactory. Such a plate conveniently contains collector channels defined by walls of 0.5mm (0.02in) width and 0.2mm (0.01in) height. The plate body is preferably 0.2mm (0.01in) thick (a plate that is significantly thicker is rather to difficult to flex into contact with the test surface, while a significantly thinner plate is not likely to be sufficiently robust to withstand the rough conditions commonly prevailing in the collector's deployment).

The guide channel structure geometry may be any suitable. Preferably, however, the point in the plate to which the collected hydrogen is delivered for onwards passage is located centrally of the plate, and thus the channels are most conveniently either radial or - and this is preferred - a single spiral terminating at the centre of the plate. The gap between adjacent grooves is advantageously about 1cm (0.4in).

The actual gas exit means is conveniently capillary piping leading off to a remote detector and pump. For a 15cm (6in) diameter plate, the gas exit capillary/plate union is preferably minimal so as not significantly to limit the collector plate's flexibility.

In use the plate is held up against the test surface - and clearly it must be held there firmly and securely for some considerable period of time sufficient to collect enough hydrogen for the purpose of the test. To assist in this, then, the collector plate is most preferably first supported on a backing plate, the supports being such as to permit the required flexing of the collector plate to occur when the combination is pushed into operative contact with a curved test surface. Thus, for example, the backing plate is conveniently a relatively rigid member to which the collector plate is attached by flexible/bendable/pivotable/floating mounting means disposed around the peripheries of both plates (leaving the centre of the collector plate free). The mounting means could be rubber pads, or springs, or some combination of these. One preferred mounting means is a combination of a support rod loosely mounted (in an oversize bush) in the backing plate, the rod carrying at its free end one end of a spring to the other end of which the collector plate is mounted. A plurality of these, spaced uniformly around the two plates' peripheries, causes the collector plate to be so supported on the backing plate that it can flex sufficiently freely both inwards and outwards and also to and fro when offered up to a curved test surface.

As noted above, in use the collector plate must be held up against the test surface firmly and securely for some time. Obviously, the backing plate can be provided with a handle, or some other means by which it can be grasped, to facilitate its utilisation, and a long handle may also be desirable to assist in locating the collector plate against a distant, hard-to-reach surface, or against some surface that is, for example, too hot to allow the Operator to get close. However, since the collector plate may need to be held in position for an hour or so it is better not to hold it there by hand but instead in some manner to clamp it in position. And while there may be other ways to attain the required end, in the present invention this clamping is most preferably achieved magnetically. Thus, most commonly the surfaces - the steel surfaces - from which hydrogen emanates are magnetic (non-magnetic steels are austenitic and effectively hydrogen impermeable), and therefore in principle a magnetic means of attachment of the collector plate is viable in many of the instances where detection or measurement of emanating hydrogen is of interest.

Magnetic clamping can be effected by placing a plurality of magnets on the back surface of the collector plate and spaced around the peripheral area; by using reasonably strong magnets, the magnetic field each produces is easily capable of extending through the plate and to the (in use) underlying test surface, so clamping the plate onto the surface indefinitely.

The magnets are advantageously retained on the collector by being mounted on the backing plate - desirably in such a manner as to afford them limited vertical and rocking movement. As noted above, the preferred mounting means for the collector plate on the backing plate is a combination of a support rod loosely mounted in the backing plate and carrying at its free end a spring on which the collector plate is mounted. If the spring bears the magnet at its free end, then the resultant plurality of these combinations, spaced uniformly around the two plates' peripheries, causes the collector plate to be so supported on the backing plate that it can as desired flex freely when offered up to a curved test surface and yet be clamped to that surface by the magnets. If the magnets are sufficiently strongly attracted to the test surface then they will bend the collector plate towards that surface (and as the plate bends, and the distance between each magnet and the test surface decreases, so the attraction between each magnet and the surface increases, facilitating further deformation of the plate until the plate is firmly attached to the steel). The spring/bush mounting enables the magnets to twist as necessary to ensure their approximately closest approach to the plate.

In practice most metal surfaces to be tested will be covered in a paint layer one or two millimetres thick, and to provide some uniformity in the magnetic forces involved it is therefore preferred to utilise very strong magnets positioned such that they cannot actually come into "direct" contact with the surface (for a strong magnet at some distance from the surface the useful attractive forces change less rapidly with distance than for a weak magnet at some shorter distance at which these forces are achieved). Conveniently, then, each magnet is itself individually mounted in a small holder such that its active end is recessed, and can never actually come into contact with the collector plate back (the holder itself will contact the plate's back, and so should therefore preferably be of a "slippery" material, such as PTFE [polytetrafluoroethylene] to assist it to move laterally across the plate's back surface as will be required as the plate flexes). In this way, very powerful magnets can be utilised, to ensure sufficient flexing of the plate round high curvature surfaces, but the plate, and its magnets, can always relatively easily be freed from the steel after the test is complete.

Notionally it is only necessary for there to be two magnets at the perimeter and extreme ends of a collector plate to provide plate deformation to a steel surface. However, in the case of attachment to steel of cylindrical symmetry, such as a pipe, clearly two magnets will only enable complete plate/surface contact if the collector is oriented such as to ensure the magnets are in the plane of the cylinder cross-section. Since it is usually preferable to ensure full plate/surface contact irrespective of collector orientation (as the operator may not be appreciative of the need for such careful orientation, for example, in being up a ladder, or in attaching the collector to the base of a pipe near the ground and therefore where the view of attachment of the collector is partially obscured) it is better to have more magnets. To enable fully acceptable test surface attachment irrespective of orientation, a minimum of six magnets is preferable, the magnets being arranged in a ring about the periphery of the collector plate, equidistant from each other (thus, generally at the points of a hexagon). The magnets are preferably disc shaped, having both their axis of cylindrical symmetry and North-South poles directed normal to the plate surface. It is also preferable for the North-South poles of all the magnets to be all parallel; this ensures mutual repulsion between the magnets, which assists their symmetrical and uniform deformation (this is preferable to, for example, the poles between any two neighbouring magnets being reversed, which would result in the magnets deforming unpredictably and with a tendency to 'pair up').

A flat collector plate of, say, 15cm (6in) diameter made to conform to a 30cm (12in) diameter pipe requires maximum plate displacement of some 6mm (0.25in) at the perimeter. A typical 0.5mm (0.02) thickness of the plate (including the groove walls), and a nominal 2.5mm (0.1in) thickness of paint on the pipework would suggest the need for a magnet facilitating plate movement at a maximum distance of some 9mm (0.35in). This is readily achieved with high power magnets of small dimensions, such as 7mm (0.27in) thickness, 14mm (0.55in) diameter, nickel-coated neodymium boron magnets whose poles are co-axial with the centre of symmetry as described above.

As noted above, each magnet is conveniently secured in a small holder - a cradle or cup - and held there either by glue, by using a circlip, or by being a push fit, and it is recessed slightly in the cup (behind a lip) so that attachment of the plate to an uncoated steel surface is not so strong as to make detachment difficult. In the case of the disc magnets mentioned above, a recess of 1mm (0.04in) in the cup is sufficient to enable excellent attachment of the plate described above to 30cm (12in) diameter steel pipework. The cup lip which contacts the plate is preferably rounded (and as noted before made of a material which readily slips against the plate), so that any angular movement of the cup necessary in securing the plate to a curved test surface is facilitated. In addition it is preferable for the cup material to be non-magnetic, tough and readily machined; PTFE is good for this purpose.

The invention provides for magnetic movement to ensure plate conformation to any reasonable test surface no matter what its curvature. The means of this movement may, as indicated hereinbefore, be any suitable

- a combination of flexible arms, ball and socket joints, springs, levers or slots, say. When using a magnet holder such as the cup discussed above, the cup's exterior is conveniently machined with a screw thread so to accommodate a spring (to enable a rocking movement) simply by the spring being "wound" onto the cup. At the other end of the spring, an end piece similarly machined to accommodate the spring is attached to an arm
- the rod - which moves through a bushed slot in the collector backplate, enabling free vertical movement. A non-extensible string between the two holder parts prevents over-stretching of the spring during detachment from a steel surface.

Finally, the collector plate may be provided with a skirt at or just beyond its perimeter to prevent in use the ingress of water between the plate and steel, or provide limited protection of the plate edge.

In use, the collector plate of the invention collects the hydrogen gas that emanates from the test surface. The flux of hydrogen from the surface ($J$: the flow per unit area) is related to the measured concentration ($C$) of hydrogen entrained in the carrier gas, the carrier gas flow ($F$), and the effective area ($A_{eff}$) over which hydrogen is measured, by the equation

$$J = (F \times C)/A_{eff} \qquad (i)$$

It is worth exploring the optimal conditions to enable meaningful measurement.

A significant flux of emanating hydrogen, $J$, is commonly very small. Specifically, in sour gas corrosion of pipework, the walls of the pipe are commonly of the order 1cm (4in) thick, and a significant flux is deemed to be of the order of a few thousand millionths of a cubic centimetre of hydrogen per square centimetre of surface per second ($pl/cm^2/s$). Thus, from Equation (i) it is preferable to measure the hydrogen concentration $C$ captured in the gas steam to as low a threshold as possible. This sensitivity limit is constrained - by the concentration of hydrogen in air (0.5ppm at sea level), and by the limits of the present technology - to about 0.1ppm hydrogen.

Next, it is preferable to minimize the carrier gas flow $F$: essentially, the lower the flow of carrier gas across the test surface the greater is the hydrogen enrichment as a given hydrogen flux is entrained into it. However, if that gas flow is too low the hydrogen in the carrier gas stream is liable to back diffuse - that is, to move against the prevailing gas stream, thus lowering Aeff for a particular collecting device. A very low flow will also significantly delay the hydrogen in its journey to the detector, particularly if the distance between the collector and detector exceeds one meter (as may be desirable in testing steel surfaces whose proximal air temperatures exceed what can be tolerated by a particular detector). A typical adequate flow for a collector of 15cm (6in) diameter is 0.5cm$^3$/s.

Finally, it is preferable to maximize Aeff. At a flow rate of 0.5cm$^3$/s, about 70% of the hydrogen emanating from a smooth flat steel surface zone circumscribed by a circle of diameter 15cm (6in) is captured by a collector of similar dimensions, thus Aeff in this case is about 100 cm$^2$. Drawing ambient air into a detector capable of resolving 0.2ppm hydrogen, the minimum resolvable flux according to equation (i) is therefore :

$$0.5 \times 0.2 \times 10^{-6} / 100 \text{ pl/cm}^2/\text{s} = 1 \text{ pl/cm}^2/\text{s}.$$

In the above, the collector is of dimensions convenient for manual manipulation, but in many instances the test surface geometry will constrain the collector's maximum size. Conversely, in one particular application, namely that relating to the testing of high-pressure hydrogen entry into reformer vessels in petrochemical production, due to the high pressures involved the steel walls are very thick - commonly 5cm (2in) as compared with 1cm (0.4in) for pipelines - so hydrogen emanation lower than 1 pl/cm$^2$/s may be significant and require a larger collector (say, of diameter 30cm (12in).

An embodiment of the invention is now described, though by way of illustration only, with reference to the accompanying diagrammatic Drawings in which:

Figure 1  shows a general view of the testing (for emanating hydrogen) of a surface in accordance with the invention;

Figures 2A-C  show views - perspective from above, side elevation, and top plan (in see-through) - of a collector device of the invention;

Figure 3  shows in side elevation the device of Figure 2B in use on a pipeline pipe; and

Figure 4  shows, in side elevation (like Figure 2B), part of a different embodiment of a device of the invention.

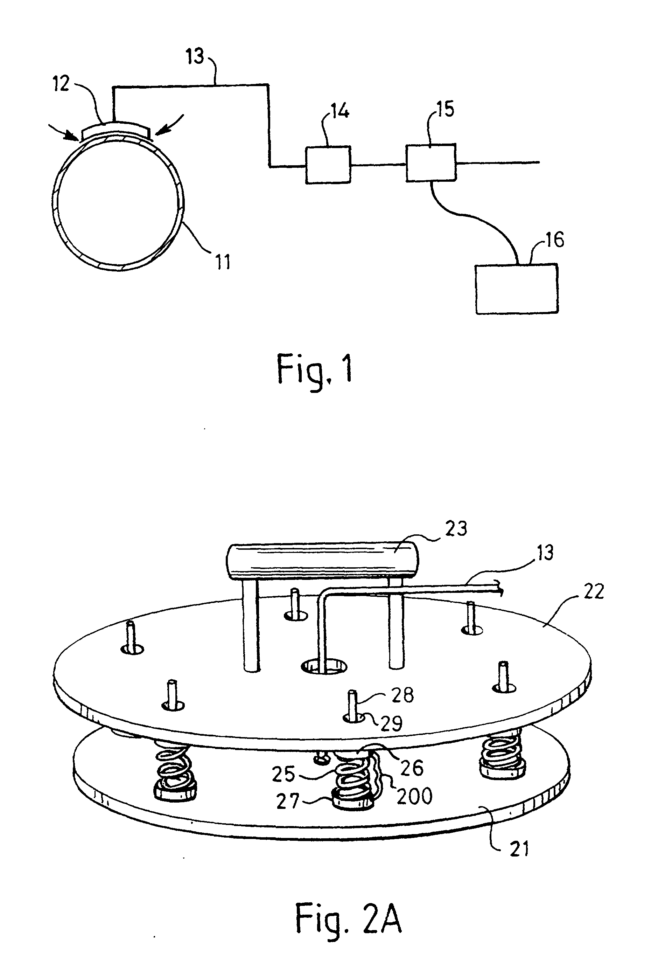

In Figure 1 there is show a section of pipeline pipe (11) onto which has been magnetically clamped a collector device (12) of the invention. Atmospheric air is drawn under and into the collector plate around its edges, and travels therethrough and along to exit *via* a narrow-bore pipe (13), carrying any emanated hydrogen with it. The air/hydrogen mixture is drawn on, by a pump (14), and then passed on to a hydrogen detector (15) and then discarded back to atmosphere. The detector's output is fed to a suitable measuring and recording device (16), and the collected data made ready for subsequent use.

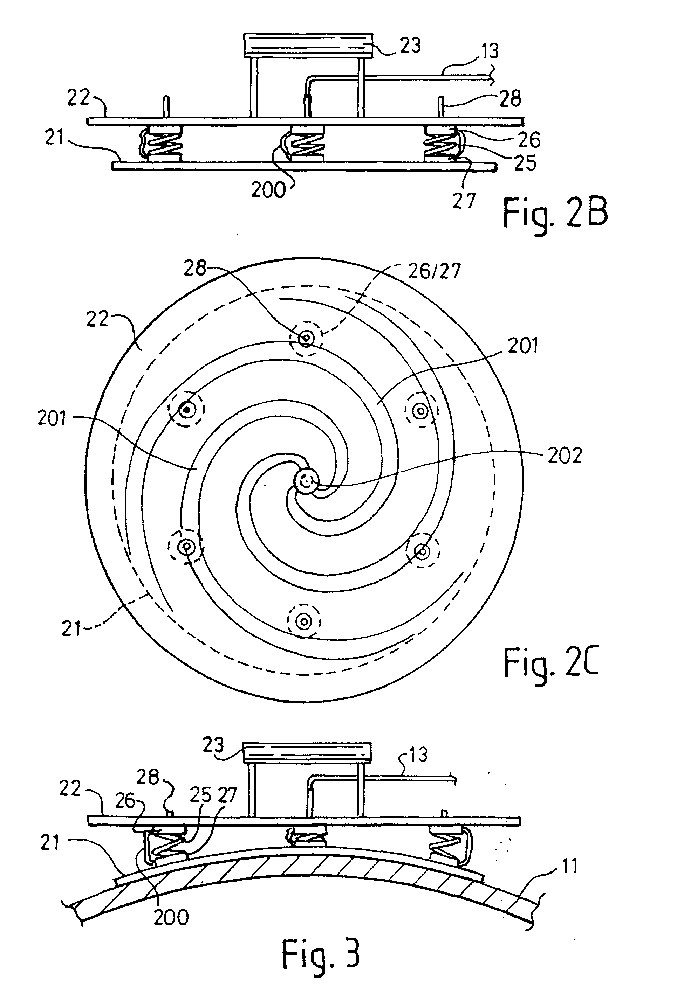

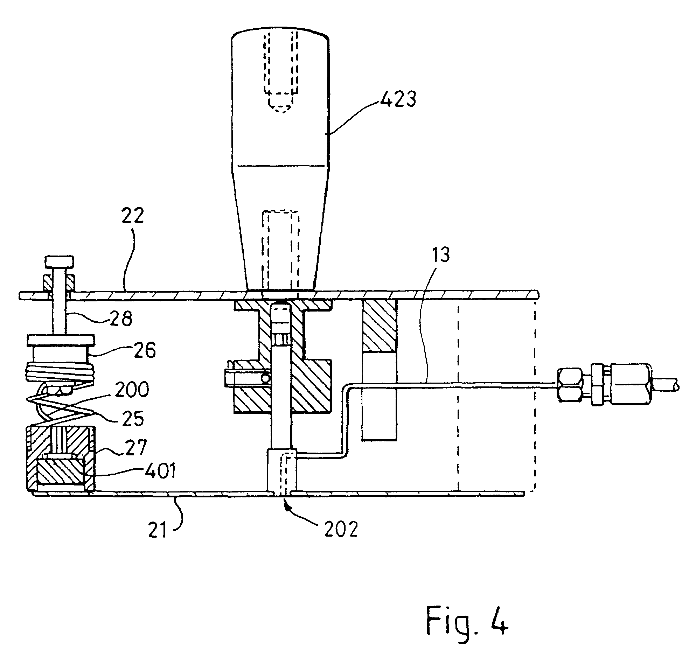

One embodiment of the collection device of the invention is shown in Figures 2A,B and C. The device comprises a collector plate (21) mounted on a backplate (22) itself bearing a carrying handle (23). There are six individual mountings (as 24) for the collector plate 21 (on the backplate 22); each is essentially a spring (25) with a holder (26,27) at each end (which is threaded so that the spring can be "wound" on to it). The upper (as viewed) holder 26 is itself supported on a rod (28) passing loosely through a bushed aperture (29) in the backplate 22; the lower (as viewed) holder 27 carries recessed therein a strong magnet (not shown in Figures 2: see Figure 4). The combination of rod 28 and spring 25 permits the bottom holder 27, and thus the magnet inside it, to within reason move up and down and side to side, as required when the device is used and the collector plate 21 flexes into contact with a test surface. An inelastic tie, or string (200) prevents the collector plate being pulled too far from the backplate 22, and so stops the springs 25 from becoming over-extended.

As shown very diagrammatically in the top plan view of Figure 2C (in which the handle 23 is *not* depicted), the underside (as viewed) of the collector plate 21 is spirally grooved so as to define channels as (201) - three are shown, and shaded to make them stand out - , bounded by low walls, along which air, and emanated hydrogen, can be drawn in from the peripheral areas to the centre point, and then, through an opening (202) connected to the pipe 13, and thence out of the collector plate 21 and on to the detector 15.

Figure 3 shows the collector device of the invention being used - as it is in Figure 1 - on a pipeline pipe (11: shown here in part only). There can clearly be seen the way in which the clamping magnets (see Figure 4) cause the collector plate 21 to flex into contact with the pipe 11, the rod and spring combination 28/25 allowing the magnets, in their holders 27, to move up and down and from side to side, so as the better to conform.

A slightly different version of the collector device of the invention is shown in Figure 4. This version has a different sort of carrying handle (423), and there can clearly be seen the magnet (401) recessed within the bottom holder 27, the rod 28 in its hole in the backplate 22, the spring 25 "wound" around the threaded top and bottom holders 26,27, and the string tie 200 between those two holders.

## Claims

1. A collector device (12) of a hydrogen collection and detection system (12-16) comprising a flexible collector plate (21) having input means for receiving a carrier gas and output means (13) for delivering a mixture of the carrier gas and emanated hydrogen operatively connectable from the output means (13) to a hydrogen detector (15); said flexible collector plate (21) having a receptor face with guide channels (201) along which said mixture of carrier gas and emanated hydrogen may be channelled and sent to said detector (15);
   **characterised in that** the body of said flexible collector plate (21) is deformable so that, in use, it conforms to a curved test surface (11) and the receptor face of the plate (21) contacts said curved test surface (11).

2. A collector device as claimed in Claim 1, wherein the collector plate (21) comprises untempered sheet stainless steel into which are formed collecting grooves (210) constituting the guide channels, and wherein the guide channel structure geometry forms a single spiral terminating at the centre (202) of the plate (21).

3. A collector device as claimed in either of the preceding Claims, wherein the collector plate (21) is supported on a backing plate (22), the supports (225-28) being such as to permit the required flexing of the collector plate (21) to occur when the combination is pushed into operative contact with a curved test surface (11), wherein the backing plate (22) is a relatively rigid member to which the collector plate (21) is attached by flexible/bendable/pivotable/floating mounting means (25-28) disposed around the peripheries of both plates, and wherein each mounting means is a combination of a support rod (28) loosely mounted in an oversize bush (26) in the backing plate (22), the rod (28) carrying at its free end one end of a spring (25) to the other end of which the collector plate (21) is mounted.

4. A collector device as claimed in Claim 3, wherein, to hold in use the collector plate (21) against the test surface (11), the plate (21) has associated clamping means (27,401), the clamping means is a plurality of magnets (401) on the back surface of the plate (21) and spaced around the peripheral area thereof, and each magnet (401) is mounted on the backing plate (22) by magnet mounting means (25-27) that affords it limited vertical and rocking movement.

5. A collector device as claimed in Claim 4, wherein each mounting means for the collector plate on the backing plate is a combination of a support rod (28) loosely mounted in the backing plate (22) and carrying at its free end a spring (25) on which the collector plate (21) is mounted, and the spring (25) bears the magnet (401) at its free end, and wherein each magnet (401) is itself individually mounted in a holder (27) such that its active end is recessed, and can never actually come into contact with the collector plate back.

6. A collector device as claimed in either of Claims 4 and 5, wherein there are at least six magnets (401), arranged in a ring about the periphery of the collector plate (21), equidistant from each other.

7. A collector device as claimed in any of Claims 4 to 6, wherein the magnets (401) are disc shaped, and are positioned with both their axis of cylindrical symmetry and North-South poles directed normal to the plate surface, and with the North-South poles of all the magnets parallel.

8. A collector device as claimed in any of the preceding Claims, wherein the collector plate (21) is provided with a skirt at or just beyond its perimeter.

9. A method of detecting hydrogen emanating from a surface, utilizing a hydrogen collection and detection device as claimed in any one of the preceding claims;
   comprising the steps of:

   a) operatively applying the hydrogen collection and detection system to a test surface (11);
   b) allowing a carrier gas to mix with hydrogen emanating from the surface;
   c) channelling the mixture of carrier gas and emanated hydrogen between the receptor face of the flexible collector plate (21) and the test surface (11) towards the output means (13);
   d) carrying the mixture via the output means (13) to a collection vessel; and
   e) performing an analysis of the mixture to determine hydrogen flux.

10. The method of Claim 9, wherein the hydrogen collection and detection device further comprises a backing plate (22) and a plurality of supports (225 - 28) for supporting the flexible collector plate (21) on the backing plate (22), mounting means (25 - 28) for attaching the flexible collector plate (21) to the backing plate (22), clamping means (27, 401) associated with the flexible collector plate (21) to hold the flexible collector plate (21) against the test surface (11), wherein the clamping means (27) comprise a plurality of magnets (401) on the surface of the flexible collector plate opposite to the receptor face.

11. The method of Claim 10, wherein the mounting means comprises a combination of a support rod (28) mounted in the backing plate (22) and carrying at a free end thereof a spring (25) on which the flexible collector plate (21) is mounted, wherein the spring (25) bears one of said plurality of magnets (401) on a free end thereof and whereby each magnet (401) is individually mounted in a holder (27).

12. The method of any one of Claims 9 to 11, wherein the guide channels (201) of the receptor face comprise a geometry that forms a single spiral terminating near the centre of the flexible collector plate (21).

**Patentansprüche**

1. Auffangvorrichtung (12) eines Wasserstoff-Auffang- und Detektorsystems (12 - 16), bestehend aus einer flexiblen Auffangplatte (21) mit Eingabemitteln zur Einleitung eines Trägergases und Ausgabemitteln (13) zur Ableitung eines Gemisches aus dem Trägergas und ausgetretenem Wasserstoff, im Betrieb zwischen den Ausgabemitteln (13) und einem Wasserstoffdetektor (15) verbindbar;
welche besagte flexible Auffangplatte (21) eine Auffangseite mit Führungskanälen (201) darin aufweist, längs welcher besagtes Gemisch von Trägergas und ausgetretenem Wasserstoff geleitet und so besagtem Detektor (15) zugeführt werden kann;
**dadurch gekennzeichnet, daß** der Körper der besagten Auffangplatte (21) verformbar ist, so daß er sich im Betrieb einer gekrümmten Prüfoberfläche (11) anpaßt, und daß die Auffangseite der Platte (21) an besagter gekrümmter Prüfoberfläche (11) anliegt.

2. Auffangvorrichtung nach Anspruch 1, worin die Auffangplatte (21) aus einem unvergüteten Edelstahlblech besteht, in welches Sammelnuten (210) eingeformt sind, welche die Führungskanäle bilden, und worin die geometrische Struktur der Führungskanäle eine einzelne Spirale formt, die im Zentrum (202) der Platte (21) ausläuft.

3. Auffangvorrichtung nach einem beliebigen der vorangehenden Ansprüche, worin die Auffangplatte (21) von einer Trägerplatte (22) getragen wird, wobei die Halterungen (225 - 28) derart sind, daß sie das erforderliche elastische Nachgeben der Auffangplatte (21) in der Weise erlauben, daß es auftritt, wenn die Kombination in Arbeitskontakt mit der gekrümmten Prüfoberfläche (11) gedrückt wird, worin die Trägerplatte (22) ein relativ starres Teil ist, an welchem die Auffangplatte (21) über flexible/biegsame/schwenkbare/schwimmend gelagerte Befestigungsmittel (25 - 28) angebracht ist, die um den Umfang der beiden Platten verteilt sind, und worin jedes Befestigungsmittel eine Kombination aus einem lose in einer übergroßen Buchse (26) an der Trägerplatte (22) eingebauten Trägerstab (28) ist, welcher Stab (28) an seinem freien Ende ein Ende einer Feder (25) trägt, an deren anderem Ende die Auffangplatte (21) befestigt ist.

4. Auffangvorrichtung nach Anspruch 3, worin, um im Gebrauch die Auffangplatte (21) gegen die Prüfoberfläche (11) zu halten, die Platte (21) ihr zugeordnete Spannmittel (27, 401) aufweist, wobei die Spannmittel von einer Vielzahl von Magneten (401) auf der Rückseite der Platte (21) gebildet werden, die im Abstand von einander über die Umfangsfläche derselben verteilt sind, und wo jeder Magnet (401) an der Trägerplatte (22) über Magnetbefestigungsmittel (25 - 27) befestigt ist, die ihm einen begrenzten vertikalen Hub und Kippfreiheit erlauben.

5. Auffangvorrichtung nach Anspruch 4, worin jedes der Befestigungsmittel für die Auffangplatte an der Trägerplatte eine Kombination aus einem Trägerstab (28) ist, der lose an der Trägerplatte (22) angebracht ist und an seinem freien Ende eine Feder (25) trägt, an welcher die Auffangplatte (21) befestigt ist, und wo die Feder (25) an ihrem freien Ende den Magneten trägt, und worin jeder Magnet (401) seinerseits individuell so in einer Halterung (27) gelagert ist, daß sein aktives Ende zurückversetzt ist und niemals effektiv mit der Rückseite der Auffangplatte in Kontakt kommen kann.

6. Auffangvorrichtung nach Anspruch 4 oder 5, worin wenigstens sechs Magnete (401) vorliegen, die in einem Ring um den Umfang der Auffangplatte (21) in gleichem Abstand von einander angeordnet sind.

7. Auffangvorrichtung nach einem beliebigen der Ansprüche 4 bis 6, worin die Magneten (401) scheibenförmig sind und so positioniert sind, daß sowohl ihre zylindrische Symmetrieachse als auch ihre Nord-Süd-Polachse senkrecht zur Plattenoberfläche ausgerichtet ist, und so daß die Nord-Süd-Polachsen aller Magneten parallel sind.

8. Auffangvorrichtung nach einem beliebigen der vorangehenden Ansprüche, worin die Auffangplatte (21) an oder knapp außerhalb ihres Umfanges mit einem umlaufenden Rand versehen ist.

9. Verfahren zur Erfassung von aus einer Oberfläche austretendem Wasserstoff unter Verwendung einer Wasserstoff-Auffang- und Detektorvorrichtung nach einem beliebigen der vorangehenden Ansprüche; folgende Schritte aufweisend:

a) betriebsfähiges Anlegen des Wasserstoff-Auffang- und Detektorsystems an eine Prüfoberfläche (11);

b) Vermischenlassen eines Trägergases mit aus der Oberfläche austretendem Wasserstoff;

c) Ableiten des Gemisches aus Trägergas und ausgetretenem Wasserstoff zwischen der Auffangseite der biegsamen Auffangplatte (21) und der Prüfoberfläche (11) bis zu den Ausgabemitteln;

d) Abführen des Gemisches über die Ausgabemittel bis zu einem Auffanggefäß; und

e) Durchführen einer Analyse des Gemisches zur Bestimmung des Wasserstoffdurchsatzes.

10. Verfahren nach Anspruch 9, worin die Wasserstoff-Auffang- und Detektorvorrichtung außerdem eine Trägerplatte (22) sowie mehrere Halterungen (225 - 28) zur Halterung der biegsamen Auffangplatte (21) auf der Trägerplatte (22) beinhaltet, sowie Befestigungsmittel (25 - 28) zur Befestigung der biegsamen Auffangplatte (21) an der Trägerplatte (22), der biegsamen Trägerplatte (21) zugeordnete Spannmittel (27, 401) zum Anlegen der Auffangplatte (21) an die Prüfoberfläche (11), und worin die Spannmittel (27) von einer Reihe von Magneten (401) an derjenigen Oberfläche der biegsamen Auffangplatte gebildet werden, welche der Aufnahmeseite gegenüberliegt.

11. Verfahren nach Anspruch 10, worin die Befestigungsmittel von einer Kombination aus einem an der Trägerplatte befestigten Trägerstab (28) gebildet werden, der an einem freien Ende desselben eine Feder (25) trägt, an welcher wiederum die Auffangplatte (21) befestigt ist, worin die Feder (25) einen der besagten mehreren Magnete (401) an einem freien Ende derselben trägt, und über welche jeder Magnet (401) einzeln in einer Halterung (27) gelagert ist.

12. Verfahren nach einem beliebigen der Ansprüche 9 bis 11, worin die Führungskanäle (201) der Auffangseite eine Geometrie aufweisen, die eine einzelne Spirale bildet, welche in der Nähe des Zentrums der biegsamen Auffangplatte (21) ausläuft.

**Revendications**

1. Un dispositif de recueil (12) d'un système de recueil et de détection d'hydrogène (12-16) comprenant une plaque de recueil flexible (21) incluant un dispositif de réception pour recevoir un gaz porteur ainsi qu'un dispositif d'émission (13) pour fournir un mélange de gaz porteur et d'hydrogène émis, pouvant être couplée en fonctionnement avec un détecteur d'hydrogène (15) à partir du dispositif d'émission (13) ; ladite plaque de recueil flexible (21) comprenant un côté de réception comportant des canaux de guidage (201) le long desquels ledit mélange de gaz porteur et d'hydrogène émis peut être conduit et acheminé audit détecteur (15) ;

**caractérisé en ce que** le corps de ladite plaque de recueil flexible (21) est déformable, de manière à ce qu'il se conforme à une surface de test courbe (11) et que le côté de réception de la plaque (21) soit au contact de ladite surface de test courbe (11) en utilisation.

2. Un dispositif de recueil selon la revendication 1, dans lequel la plaque de recueil (21) comprend un feuillet d'acier inoxydable non - trempé dans lequel des rainures de recueil (210) sont pratiquées qui constituent les canaux de guidage, et dans lequel la géométrie de la structure des canaux de guidage forme une spirale unique terminant au centre (202) de la plaque (21).

3. Un dispositif de recueil selon l'une quelconque des revendications précédentes, dans lequel la plaque de recueil (21) est disposé sur une plaque de soutien (22), les supports (225-28) étant tels qu'ils permettent la flexibilité requise de la plaque de recueil (21) lorsque la combinaison est poussée pour utilisation au contact d'une surface de test courbe (11), dans lequel la plaque de soutien (22) est un membre relativement rigide auquel la plaque de recueil (21) est attachée par des dispositifs de fixation flexibles / pliants / pivotants / flottants (25-28) disposés autour des périphéries des deux plaques, et dans lequel chaque dispositif de fixation comprend une combinaison d'une barre de soutien (28) lâchement disposée dans une bague surdimensionnée (26) dans la plaque de soutien (22), la barre (28) portant à son extrémité libre une extrémité d'un ressort (25), à l'autre extrémité duquel la plaque de recueil (21) est fixée.

4. Un dispositif de recueil selon la revendication 3, dans lequel la plaque (21) comprend un dispositif de verrouillage (27, 401) associé pour maintenir la plaque de recueil (21) contre la surface de test (11) pour son utilisation, le dispositif de verrouillage comprenant plusieurs aimants (401) sur la surface arrière de la plaque (21) qui sont espacés autour de sa périphérie, et chaque aimant (401) étant disposé sur la plaque de soutien (22) par un dispositif de fixation d'aimant (25 - 27) lui permettant un mouvement vertical et oscillant limité.

5. Un dispositif de recueil selon la revendication 4, dans lequel chaque dispositif de fixation sur la plaque de soutien pour la plaque de recueil comprend une combinaison d'une barre de soutien (28) lâchement disposée dans la plaque de soutien (22) et portant un ressort (25) à son extrémité libre sur lequel la plaque de recueil (21) est fixée, le ressort (25) portant l'aimant (401) à son extrémité libre, et dans lequel chaque aimant (401) est lui-même individuellement logé dans un étau (27), de manière à ce que son extrémité active soit encastrée et ne puisse jamais entrer en contact avec le dos de la plaque de recueil.

6. Un dispositif de recueil selon l'une quelconque des revendications 4 ou 5, dans lequel il y a au moins six aimants (401) disposés en cercle autour de la périphérie de la plaque de recueil (21), qui équidistants l'un par rapport à l'autre.

7. Un dispositif de recueil selon l'une quelconque des revendications 4 à 6, dans lequel les aimants (401) ont la forme d'un disque, et sont disposés avec leur axe de symétrie cylindrique ainsi que leur pôle Nord-Sud en direction normale par rapport à la surface de la plaque, ainsi qu'avec les pôles Nord-Sud de tous les aimants parallèles.

8. Un dispositif de recueil selon l'une quelconque des revendications précédentes, dans lequel la plaque de recueil (21) est configurée avec une jupe disposée à son périmètre ou juste au-delà de son périmètre.

9. Une méthode de détecter l'hydrogène émanant d'une surface, utilisant un dispositif de recueil et de détection d'hydrogène selon l'une quelconque des revendications précédentes ; comprenant les étapes de :

> a) appliquer le système de recueil et de détection d'hydrogène à une surface de test (11) en opération ;
> b) permettre à un gaz porteur de se mélanger avec l'hydrogène émanant de la surface ;
> c) acheminer le mélange de gaz porteur et d'hydrogène émis entre le côté de réception de la plaque de recueil flexible (21) et la surface de test (11) vers le dispositif d'émission (13) ;
> d) transporter le mélange au moyen du dispositif d'émission 13 jusqu'à un réservoir de recueil ; et
> e) pratiquer une analyse du mélange pour déterminer le fluide hydrogène.

10. Une méthode selon la revendication 9, dans laquelle le dispositif de recueil et de détection d'hydrogène comprend en outre une plaque de soutien (22) et plusieurs supports (225-28) pour soutenir la plaque de recueil flexible (21) sur la plaque de soutien (22), un dispositif de fixation (25 - 28) pour attacher la plaque de recueil flexible (21) à la plaque de soutien (22), un dispositif de verrouillage (27, 401) associé avec la plaque de recueil flexible (21) pour maintenir la plaque de recueil flexible (21) contre la surface de test (11), le dispositif de verrouillage (27) comprenant plusieurs aimants (401) sur la surface de la plaque de recueil flexible opposée au côté de réception.

11. Une méthode selon la revendication 10, dans laquelle le dispositif de fixation comprend une combinaison d'une barre de soutien (28) disposée dans la plaque de soutien (22) et portant un ressort (25) à son extrémité libre sur lequel la plaque de recueil (21) est fixée, le ressort (25) portant un desdits plusieurs aimants (401) à son extrémité libre, et chaque aimant (401) étant individuellement logé dans un étau (27).

12. Une méthode selon l'une quelconque des revendications 9 à 11, dans laquelle les canaux de guidage (201) du côté de réception présentent une géométrie formant une spirale unique qui se termine à proximité du centre de la plaque de recueil flexible (21).

Fig. 1

Fig. 2A

Fig. 2B

Fig. 2C

Fig. 3

Fig. 4